**Europäisches Patentamt**

(19) **European Patent Office**

**Office européen des brevets**

(11) Veröffentlichungsnummer: **0 115 299**
**B1**

(12) # EUROPÄISCHE PATENTSCHRIFT

(45) Veröffentlichungstag der Patentschrift:
21.08.85

(51) Int. Cl.⁴: **C 07 C 37/86,** C 07 C 39/15,
C 07 C 37/04

(21) Anmeldenummer: **84100524.2**

(22) Anmeldetag: **19.01.84**

(54) Verfahren zur gleichzeitigen Gewinnung von 4-Hydroxydiphenyl und 4,4'-Dihydroxydiphenyl.

(30) Priorität: **01.02.83 DE 3303220**

(43) Veröffentlichungstag der Anmeldung:
**08.08.84 Patentblatt 84/32**

(45) Bekanntmachung des Hinweises auf die Patenterteilung:
**21.08.85 Patentblatt 85/34**

(84) Benannte Vertragsstaaten:
**BE CH DE FR GB IT LI**

(56) Entgegenhaltungen:
**US - A - 1 885 176**
**US - A - 3 993 700**
**US - A - 4 366 329**

**CHEMICAL ABSTRACTS, Band 92, Nr. 5, 4. Februar
1980, Seite 747, Nr. 41563d, Columbus, Ohio, USA**

(73) Patentinhaber: **BAYER AG, Konzernverwaltung RP
Patentabteilung, D-5090 Leverkusen 1 Bayerwerk (DE)**

(72) Erfinder: **Gabel, Eike, Dr., Andreas-Gryphius-Strasse 20,
D-5000 Köln 80 (DE)**
Erfinder: **Blank, Heinz Ulrich, Dr., Am Geusfelde 35,
D-5068 Odenthal (DE)**
Erfinder: **Behre, Horst, Dr., Zur alten Linde 12,
D-5068 Odenthal (DE)**

**0 115 299**

**Beschreibung**

Die vorliegende Erfindung betrifft ein Verfahren zur gleichzeitigen Gewinnung von 4-Hydroxydiphenyl (Mono-OD) und 4,4'-Dihydroxydiphenyl (DOD) aus einem Gemisch, das die beiden Stoffe enthält, durch Behandlung eines solchen Gemisches mit wäßriger Alkalilauge.

Dihydroxy-diphenyle werden beispielsweise als Ausgangsmaterial für hochwertige Kondensationspolymere, wie Polycarbonate, Polyester und Pulverlacke verwendet, wobei besonders die Eigenschaft der Hochtemperatur-Beständigkeit bemerkenswert ist (DE-OS 30 31 094). Als Kettenabbrecher zur Einstellung der Molekulargewichte sind wegen der strukturellen Ähnlichkeit Monohydroxydiphenyle einsetzbar. Dihydroxy-diphenyle finden ferner Verwendung als Zwischenprodukte für pharmazeutische Produkte sowie als Stabilisatoren und Antioxidantien für Kautschuke, Öle und Polymere. 4-Hydroxy-diphenyle (p-Phenyl-phenole) werden als Zwischenprodukte zur Herstellung von Lackharzen, nicht-ionogenen Emulgatoren und Planzenschutzmitteln verwendet (Ullmanns Enzyklopädie der technischen Chemie, 4. Auflage, Band 18, Seite 219).

Ein wichtiger Herstellungsweg für Mono-OD und DOD geht aus von den Diphenyl-4-sulfonsäure bzw. der Diphenyl-4,4'-disulfonsäure, die mit Hilfe einer Alkalischmelze in Mono-OD bzw. DOD unter Austausch der Sulfogruppe gegen die Hydroxygruppe umgesetzt werden (DE-OS 30 31 094). Zu dieser Alkalischmelze werden vielfach die Natriumsalze der genannten Sulfonsäuren eingesetzt, die nach der Sulfonierung von Diphenyl mit überschüssiger Schwefelsäure, Verdünnen des Sulfonierungsansatzes mit Wasser, partieller Neutralisation mit Natronlauge oder Aussalzen mit Kochsalz, anschließender Filtration und Trocknung gewonnen werden.

Während DOD auf dem genannten Wege so hergestellt werden kann, daß keine oder nur geringe Mengen an Mono-OD abgetrennt werden müssen, läßt sich bei der Herstellung von Mono-OD die Sulfonierung von Diphenyl wirtschaftlich nur so durchführen, daß eine beträchtliche Menge an Diphenyldisulfonsäure neben der gewünschten Diphenyl-monosulfonsäure entsteht.

Es hat daher nicht an Versuchen gefehlt, die beiden genannten Stoffe in reiner oder zumindest angereicherter Form zu erhalten. Industriell nutzbare Methoden zur Reingewinnung von Stoffen oder zur Trennung von Substanzgemischen müssen sich durch eine einfache Durchführbarkeit bei gleichzeitig hoher Selektivität der Trennung auszeichnen. Zu solchen Trennmethoden gehören beispielsweise die Destillation oder Rektifikation, die Sublimation, die Flüssig-Flüssig-Extraktion, die Kristallisation aus Lösung oder Schmelze und Diffusionstrennverfahren. Die Anwendung solcher Trennverfahren auf eine Trennung und Gewinnung von Mono-OD und DOD wird jedoch durch die Ähnlichkeit dieser beiden Verbindungen in ihren chemischen und physikalischen Eigenschaften begrenzt. Ebenso stehen ungünstige Eigenschaften dieser Substanzen bestimmten Trennmethoden im Wege; so ist beispielsweise aufgrund der ungünstig hohen Sublimations- und Schmelzpunkte eine Trennung durch Sublimation, Destillation oder Kristallisation aus der Schmelze schwierig. Aufgrund dieser Schwierigkeiten ist daher trotz des lange bestehenden Wunsches nach Reingewinnung der genannten Stoffe die Zahl der in der Literatur vorgeschlagenen Trennverfahren gering.

In der JP-Patentanmeldung 56-92236 (1981) wird beispielsweise vorgeschlagen, Mono-OD und DOD dadurch zu trennen, daß eine wäßrige Lösung dieser Phenole in Alkalilauge mit einem nicht mit Wasser mischbaren Alkohol oder Keton bei erhöhtem Druck extrahiert wird, wobei Mono-OD als freies Phenol in die organische Phase übergeht. Bereits kleine Mengen an Mono-OD erfordern hierzu eine beträchtliche Menge an organischem Lösungsmittel, so daß dieses Verfahren unwirtschaftlich und für Gemische mit großem Anteil an Mono-OD nicht anwendbar ist.

In der DE-OS 31 07 473 (Äquivalent GB 2 071 090) wird vorgeschlagen, Mono-OD aus einer alkalischen Lösung der genannten Phenolate in Wasser durch Adsorption an Aktivkohle zu entfernen. Auch hier können nur geringe Mengen an Mono-OD entfernt werden; für eine präparative Gewinnung dieses abgetrennten Mono-OD werden überhaupt keine Angaben gemacht.

Aus der JP-Patentanmeldung 54-112 844 (1979) ist es weiterhin bekannt, die Alkalischmelze von Diphenyl-sulfonsäure zum Mono-OD so zu lenken, daß das hierzu notwendige überschüssige Natriumhydroxid durch Zugabe von möglichst wenig Wasser in Lösung gebracht wird, um es von dem Mono-OD abzutrennen und gegebenenfalls nach Aufkonzentrierung wieder in die Alkalischmelze zurückzuführen. Dieses Verfahren wird in gleicher Weise benutzt, wenn aus der Diphenyl-4,4'-disulfonsäure das DOD gewonnen werden soll, wobei ebenfalls eine möglichst hochkonzentrierte Natronlauge vom zurückbleibenden DOD abgetrennt und recyclisiert wird. Hierbei liegt jedoch stets Mono-OD bzw. DOD einzeln vor, eine Trennung der Komponenten findet nicht statt.

Da es bislang keine befriedigende Trennungsmethode auf der Stufe der Hydroxydiphenyle gab, wurde auch eine Trennung auf der Stufe der Diphenyl-sulfonsäuren versucht. Solche Trennungen wurden beschrieben über die Kupfer- bzw. Natriumsalze in Kombination mit einer Trennung der freien Sulfonsäuren (Gazz. Chim. Ital. 78, 435—440 [1948]) oder die Ammoniumsalze (CS-Patent 181 835), wobei die Salze der Diphenyl monosulfonsäure von den Salzen der Diphenyl-disulfonsäure getrennt werden. Wegen der relativ guten Löslichkeit der Salze dieser Sulfonsäuren sind solche Trennverfahren jedoch verlustreich und daher für eine technische Anwendung nachteilig.

Die erwähnten Trennverfahren auf der Stufe der Sulfonsäuren erfordern zwischen der Sulfonierung von Diphenyl und der Alkalischmelze der Sulfonsäuren einen zusätzlichen Verfahrensschritt, bei dem

2

außerdem organisches Material in Form von Diphenyl-sulfonsäure bzw. Diphenyldisulfonsäure mit dem Abwasser verlorengeht und dieses Abwasser gleichzeitig belastet. Voraussetzung für die Gewinung von Mono-OD und DOD, das diese Nachteile nicht aufweist, ist daher eine Trennung auf der Stufe der Hydroxy-diphenyle.

Vor dem Hintergrund des bisherigen Wissens war es daher überraschend und nicht vorhersehbar, daß Verfahrensbedingungen gefunden werden konnten, die nicht nur eine Reinigung bzw. Anreicherung von Mono-OD bzw. DOD ermöglichen, sondern die beide Stoffe gleichzeitig in reiner bzw. angereicherter Form gewinnen lassen.

Es wurde ein Verfahren zur gleichzeitigen Gewinnung von 4-Hydroxydiphenyl (Mono-OD) und 4,4'-Dihydroxydiphenyl (DOD) aus einem die beiden Stoffe in freier Form oder in Form der Alkaliphenolate enthaltenden Gemisch gefunden, das dadurch gekennzeichnet ist, daß man ein solches Gemisch mit einer solchen Menge wäßriger Alkalilauge behandelt, daß nach gegebenenfalls erforderlicher Bildung der Alkaliphenolate 1—15 Mol Alkalihydroxid pro Mol der Diphenyl-Verbindungen vorhanden sind und eine Konzentration von 4—25 Gew.-% Alkalihydroxid, bezogen auf die Menge des vorhandenen Wassers, gegeben ist, bei einer Temperatur von −28°C bis +40°C die entstandene Lösung, die im wesentlichen das DOD enthält, vom entstandenen Bodenkörper, der im wesentlichen das Mono-DOD enthält, trennt und Lösung und Bodenkörper getrennt zur Bildung des freien Mono-OD und des freien DOD ansäuert.

Als wäßrige Alkalilauge sei die wäßrige Lösung des Hydroxids eines Alkalimetalls, bevorzugt des Natriums oder des Kaliums, erwähnt. Selbstverständlich können auch Gemische von Alkalihydroxiden eingesetzt werden. Erfindungsgemäß werden Gemische von Mono-OD und DOD mit soviel überschüssiger Alkalilauge versetzt, daß pro Mol eingesetzte Diphenyl-Verbindung in Form des Mono-OD oder des DOD 1—15 Mol, bevorzugt 3—10 Mol, des Alkalihydroxids vorhanden sind. Für den Fall, daß das Mono-OD und das DOD in Form der freien Phenole eingesetzt werden, muß das zur Bildung der Phenolate erforderliche Alkalihydroxid zusätzlich eingesetzt werden. Die Konzentration des Alkalihydroxids beträgt erfindungsgemäß, gegebenenfalls nach einer erforderlichen Bildung der Alkaliphenolate, 4—25, bevorzugt 8—20, besonders bevorzugt zwischen 8 und 15 Gew.-% Alkalihydroxid, bezogen auf die Menge des im Reaktionsgemisch vorhandenen Wassers.

Die erfindungsgemäße Behandlung des Mono-OD und DOD enthaltenden Gemisches mit wäßriger Alkalilauge wird bei normaler bis erhöhter Temperatur, beispielsweise bei 15—100°C, durchgeführt. Auch Temperaturen außerhalb dieses Bereiches können angewandt werden.

Das Reaktionsgemisch wird sodann bei einer Temperatur von −28°C bis +40°C, bevorzugt −15°C bis +35°C, besonders bevorzugt −10°C bis +30°C, getrennt. Hierbei erhält man einen Bodenkörper, der im wesentlichen das Mono-OD enthält, und eine Lösung, die im wesentlichen das DOD enthält. Die Trennung erfolgt in bekannter Weise, beispielsweise durch Filtration oder durch Zentrifugieren.

Im Filtrationsrückstand befinden sich bis zu 99% des eingesetzten Mono-OD, welches, bezogen auf 100 Gew.-Teile, beispielsweise 5, in vielen Fällen weniger als 1 Gew.-Teil DOD enthält. Falls von einem Gemisch ausgegangen wird, das bereits nur noch nahe 1 Gew.-Teil DOD vor Einsatz in das erfindungsgemäße Verfahren enthalten hatte, kann der Gehalt an DOD im erfindungsgemäß erhaltenen Filtrationsrückstand kleiner als 0,5 Gew.-Teile pro 100 Gew.-Teile Mono-OD betragen. Die Reinheit des Filtrationsrückstandes kann beispielsweise durch Waschen mit einer Alkalilauge noch weiter gesteigert werden, wobei deren Konzentration bevorzugt die gleiche ist wie die Konzentration der erfindungsgemäß zur Behandlung eingesetzten Alkalilauge ist. Falls eine besonders hohe Reinheit des im Filtrat befindlichen DOD erwünscht ist, so läßt sich dies dadurch erreichen, daß man die Löslichkeit des Mono-OD-Alkalisalzes gering hält, beispielsweise durch Erhöhung der Konzentration der Alkalilauge oder durch Absenken der Filtrationstemperatur. Das so gewonnene Mono-OD wird dann in stärkerem Maße mit DOD verunreinigt sein können.

Das Filtrat enthält 70 bis 95 Gew.-% der eingesetzten freien Alkalilauge. Es enthält ferner den Rest des eingesetzten DOD und geringe Mengen an Mono-OD, die beispielsweise 25% der Summe aus DOD und Mono-OD in der Lösung nicht übersteigen. Falls man ein Mono-OD abtrennen will, das nahezu DOD-frei ist, und hierzu beispielsweise den Ansatz stärker verdünnt oder den Rückstand mit Alkalilauge wäscht, kann der Mono-OD-Anteil an der Summe Mono-OD und DOD in der Lösung auch 25% übersteigen. Mit dem aus dem Filtrat gewonnenen Produkt, läßt sich ohne weiteres eine weitere Auftrennung durchführen. In vielen Fällen jedoch beträgt der Anteil des Mono-OD weniger als 10%, bezogen auf die Gesamtmenge DOD und Mono-OD in der Lösung. Selbst in extremen Fällen, in denen neben einem überwiegenden Anteil von Mono-OD im Ausgangsprodukt nur sehr wenig DOD vorliegt, wird im Filtrat eine beträchtliche Anreicherung des DOD um das Mehrfache seines Prozentsatzes im Ausgangsprodukt erzielt, beispielsweise läßt sich ein Ausgangsgemisch mit 11% DOD bezogen auf die Summe Mono-OD und DOD, so trennen, daß das gewonnene Filtrat 94% DOD bezogen auf die Summe Mono-OD und DOD enthält.

Im erfindungsgemäßen Verfahren können Gemische aus Mono-OD und DOD in freier Form oder in Form ihrer Alkalisalze im Verhältnis von 1 : 100 bis 100 : 1, bevorzugt 5 : 100 bis 100 : 5, besonders bevorzugt 10 : 100 bis 100 : 10 eingesetzt werden.

Die erfindungsgemäß einsetzbaren und zur gleichzeitigen Gewinnung von Mono-OD und DOD aufzutrennenden Gemische können weiterhin Alkalisulfat, beispielsweise 0 bis 5 Mol Alkalisulfat pro 1 Mol vorliegende Diphenyl Verbindungen, und gegebenenfalls weiterhin Alkalisulfit, beispielsweise 0 bis 1 Mol Alkalisulfit pro Mol vorliegende Phenolatgruppe, enthalten. Grundsätzlich ist auch ein Gehalt von mehr als 1 Mol Alkalisulfit pro Mol Phenolatgruppe für die erfolgreiche Durchführung des erfindungsgemäßen Verfahrens unschädlich. Im allgemeinen beträgt jedoch die Menge von gegebenenfalls vorhandendem Alkalisulfit etwa 1 Mol pro Mol Phenolatgruppe, da dies die Menge ist, die bei der Alkalischmelze von Sulfonaten zu den entsprechenden Phenolaten erhalten wird.

Damit ist gleichzeitig die Möglichkeit gegeben, Gemische in das erfindungsgemäße Verfahren einzusetzen, die aus der Alkalischmelze der entsprechenden Diphenyl-sulfonsäuren anfallen. In solchen technischen Gemischen befindet sich im allgemeinen eine ausreichende Menge an Alkalihydroxid, so daß es ausreicht, solchen Gemischen aus der Alkalischmelze lediglich Wasser zuzusetzen, um die erfindungsgemäßen Konzentrations- und Mengenverhältnisse einzustellen. Dieser Einsatz technischer Gemische aus der Alkalischmelze von Diphenyl-sulfonsäuresalzen stellt daher eine bevorzugte Variante des erfindungsgemäßen Verfahrens dar.

Zur Gewinnung von freiem Mono-OD bzw. freiem DOD werden der Filtrationsrückstand nach Auflösen in Wasser bzw. das Filtrat angesäuert. Hierzu wird ein pH-Wert von höchstens 8, beispielsweise 7 bis 1, eingestellt. Das Ansäuern kann grundsätzlich mit einer beliebigen Säure durchgeführt werden, deren Säurestärke größer ist als die der phenolischen Gruppen des Mono-OD bzw. des DOD.

Aus wirtschaftlichen Erwägungen wird man jedoch im allgemeinen einfache und billige Mineralsäuren, wie Salzsäure, Salpetersäure oder Phosphorsäure, bevorzugt Salzsäure oder Schwefelsäure, wählen.

Für den Fall, daß bei höheren Reinheitsanforderungen an das Mono-OD bzw. DOD weitere Reinigungsschritte erforderlich sind, lassen diese sich an das erfindungsgemäße Verfahren anschließen oder in dasselbe einbauen. Beispielsweise seien hierzu erwähnt: Anschließende Kristallisation aus organischen Lösungsmitteln, nochmaliges Umfällen der Phenolate mit Mineralsäuren, Klärung der Phenolatlösungen mit Aktivkohle (diese letztere Reinigungsmöglichkeit beispielsweise auch innerhalb des erfindungsgemäßen Verfahrens vor dem Ausfällen mit einer Säure), bei sehr hohen Reinheitsanforderungen auch Feindestillation im Hochvakuum oder Sublimation, gegebenenfalls unter vermindertem Druck.

Das erfindungsgemäße Verfahren weist folgende Vorteile auf:

1. Die Trennung und gleichzeitige Gewinnung von Mono-OD und DOD ist für einen weiten Bereich von Gemischen aus Mono-OD und DOD anwendbar.
2. Die Trennung erfolgt trotz großer Ähnlichkeit der beiden Verbindungen in hoher Selektivität. Dabei ist es, wie oben aufgeführt wurde, möglich, die Selektivität durch Wahl der Bedingungen im Rahmen des erfindungsgemäßen Verfahrens so zu beeinflussen, daß entweder die eine oder die andere der beiden Verbindungen in besonders hoher Reinheit gewonnen werden kann.
3. Bei der erfindungsgemäßen gleichzeitigen Gewinnung von Mono-OD und DOD werden Substanzverluste weitgehend vermieden.
4. Das erfindungsgemäße Verfahren wird in einem wäßrigen Medium durchgeführt und erfordert nur geringen apparativen Aufwand; es ist somit technisch einfach und wirtschaftlich durchführbar.
5. Das erfindungsgemäße Verfahren läßt sich in den technisch üblichen Prozeß zur Herstellung von Hydroxy-diphenylen, gekennzeichnet durch Sulfonierung von Diphenyl, Überführung der Sulfonsäuren in die Alkalisalze und abschließende Alkalischmelze zwanglos eingliedern.

Insbesondere der zuletzt erwähnte Vorteil ermöglicht neuartige Wege zur Herstellung von Hydroxy-diphenyl-Verbindungen. Durch die dem Fachmann geläufigen Variationen der Reaktionsbedingungen bei der Sulfonierung von Diphenyl mit Schwefelsäure (durch geeignete Wahl der Reaktionstemperatur, des molaren Verhältnisses $H_2SO_4$ zu Diphenyl, der Schwefelsäurekonzentration und/oder der Reaktionszeit) lassen sich bei weitgehend vollständigem Umsatz an Diphenyl beliebige Gemische aus Diphenyl-sulfonsäure und Diphenyl-disulfonsäure herstellen. Solche Gemische der Diphenyl-sulfonsäure lassen sich durch Alkalischmelze direkt in die entsprechenden Gemische der Alkalisalze der Hydroxy-diphenyl-Verbindungen überführen und in erfindungsgemäßer Weise zur gleichzeitigen Gewinnung von Mono-OD und DOD trennen.


### Beispiel 1

Eine Mischung aus 85,1 g 4-Hydroxydiphenyl und 93,1 g 4,4'-Dihydroxydiphenyl wurde in 1633 g 13,5 gew.-%iger Natronlauge unter Erwärmung gelöst. Nach Abkühlung auf 20°C entstand ein kristalliner Niederschlag, der abgesaugt wird.

Eine nachfolgende Analyse mittels Hochdruckflüssigchromatographie zeigte an, daß im Rückstand 76,8 g 4-Hydroxydiphenyl und 3,3 g 4,4'-Dihydroxydiphenyl und im Filtrat 88,3 g 4,4'-Dihydroxydiphenyl und 3,4 g 4-Hydroxydiphenyl enthalten waren.

## 0 115 299

Beispiele 2—4

Der Versuch aus Beispiel 1 wurde wiederholt mit den in Tabelle 1 angegebenen Veränderungen und Ergebnissen.

Tabelle 1

| Bei-spiel | Einsatzgemisch | | Alkalilauge | | im Rückstand | | im Filtrat | |
|---|---|---|---|---|---|---|---|---|
| | 4-Hydroxy-diphenyl (g) | 4,4'-Dihy-droxydi-phenyl (g) | Menge (g) | Konzentr. (Gew.-%) | 4-Hydroxy-diphenyl (g) | 4,4'-Dihy-droxydi-phenyl (g) | 4-Hydroxy-diphenyl (g) | 4,4'-Di-hy-droxy-diphenyl (g) |
| 2 | 119,2 | 55,9 | 2029 (NaOH) | 12,4 | 116,0 | 1,8 | 3,2 | 54,1 |
| 3 | 153,2 | 18,6 | 2024 (NaOH) | 12,1 | 149,7 | 0,5 | 3,5 | 18,1 |
| 4 | 153,2 | 18,6 | 1442 (NaOH) | 23,7 | 149,4 | 1,7 | 3,8 | 16,9 |

Beispiel 5

Ein Gemisch aus

234,0 g Diphenyl-4-sulfonsäure-Na-Salz,
30,8 g Diphenyl-4,4'-disulfonsäure-$Na_2$-Salz,
101,4 g $Na_2SO_4$ und
3,8 g $H_2O$,

das 1 Mol Diphenyl-Einsatz enthielt, sowie

280 g NaOH 100%ig und
168 g $H_2O$

wurden in einem Nickelautoklaven für 15 Stunden auf 320°C bei dem sich einstellenden Eigendruck von 37 bar erhitzt. Der Ansatz wurde mit 1546 g $H_2O$ verdünnt und bei 20°C filtriert.

Der Natronlauge-feuchte Rückstand wurde in 3000 ml $H_2O$ gelöst und mit HCl bis zum pH-Wert 7 neutralisiert. Nach Abkühlen auf Raumtemperatur wurde abgesaugt und mit Wasser salzfrei gewaschen. Analog wurde auch das Filtrat neutralisiert. Die abgesaugten Produkte wurden bei 70°C im Vakuumtrockenschrank getrocknet.

Man erhielt:

153,2 g Produkt I aus dem Filterrückstand mit:

99,6 Gew.-% 4-Hydroxydiphenyl[1]) = 89,7% der Theorie, bezogen auf Diphenyl
0,1 Gew.-% 4,4'-Dihydroxydiphenyl[2]) = 0,1% der Theorie, bezogen auf Diphenyl
0,3 Gew.-% Diphenyl-4-sulfonsäure = 0,2% der Theorie, bezogen auf Diphenyl

[1]) im folgenden mit Mono-OD abgekürzt;
[2]) im folgenden mit DOD abgekürzt

und

14,7 g Produkt II aus dem Filtrat mit:

8,6 Gew.-% Mono-OD = 0,75% der Theorie, bezogen auf Diphenyl
91,4 Gew.-% DOD = 7,2% der Theorie, bezogen auf Diphenyl.

5

### Beispiele 6–7

Der Versuch aus Beispiel 5 wurde wiederholt mit dem Unterschied, daß der Ansatz insgesamt mit 3475 ml bzw. mit 1095 ml $H_2O$ verdünnt wird.

Tabelle 2

| Beispiel | ml $H_2O$ zur Aufarb. | Zusammensetzung Gew.-% | | | |
| | | Produkt I | | Produkt II | |
| | | Mono-OD | DOD | Mono-OD | DOD |
|---|---|---|---|---|---|
| 6 | 3475 | 99,9 | 0,1 | 52,0 | 48,0 |
| 7 | 1095 | 94,7 | 5,2 | 0,9 | 99,1 |

### Beispiel 8

Der Versuch aus Beispiel 5 wird wiederholt, wobei folgendes Gemisch in die Alkalischmelze eingesetzt wird:

210 g Diphenyl-4-sulfonsäure-Na-Salz,
72 g Diphenyl-4,4'-sulfonsäure-$Na_2$-Salz,
111 g $Na_2SO_4$,
265 g NaOH und
176 g $H_2O$.

Nach der Reaktion wurden 1300 ml Wasser zugesetzt und die Trennung durchgeführt. Die nach Aufarbeitung gewonnenen Produkte sind in Tabelle 3 beschrieben.

### Beispiel 9

Der Versuch aus Beispiel 5 wird wiederholt, wobei folgendes Gemisch in die Alkalischmelze eingesetzt wird:

64 g Diphenyl-4-sulfonsäure-Na-Salz,
268 g Diphenyl-4,4'-disulfonsäure-$Na_2$-Salz,
320 g $Na_2SO_4$,
437 g NaOH und
293 g $H_2O$.

Nach der Reaktion wurden 2350 ml Wasser zugesetzt und die Trennung durchgeführt. Die nach Aufarbeitung gewonnenen Produkte sind in Tabelle 3 beschrieben.

Tabelle 3

| Beispiel | Zusammensetzung (Gew.-%) | | | |
| | Produkt I | | Produkt II | |
| | Mono-OD | DOD | Mono-OD | DOD |
|---|---|---|---|---|
| 8 | 99,5 | 0,5 | 5,2 | 94,8 |
| 9 | 93,5 | 6,5 | 3,3 | 96,7 |

## Patentansprüche

1. Verfahren zur gleichzeitigen Gewinnung von 4-Hydroxydiphenyl (Mono-OD) und 4,4'-Dihydroxydiphenyl (DOD) aus einem die beiden Stoffe in freier Form oder in Form der Alkaliphenolate enthaltenden Gemisch, dadurch gekennzeichnet, daß man ein solches Gemisch mit einer solchen Menge wäßriger Alkalilauge behandelt, daß nach gegebenenfalls erforderlicher Bildung der Alkaliphenolate 1—15 Mol Alkalihydroxid pro Mol der Diphenyl-Verbindungen vorhanden sind und eine Konzentration von 4—25 Gew.-% Alkalihydroxid, bezogen auf die Menge des vorhandenen Wassers, gegeben ist, bei einer Temperatur von −28"C bis +40"C die entstandene Lösung, die im wesentlichen das DOD enthält, vom entstandenen Bodenkörper, der im wesentlichen das Mono-OD enthält, trennt und Lösung und Bodenkörper getrennt zur Bildung des freien Mono-OD und des freien DOD ansäuert.

2. Verfahren nach Anspruch 1, dadurch gekennzeichnet, daß 3—10 Mol Alkalihydroxid pro Mol Diphenyl-Verbindung vorhanden sind.

3. Verfahren nach Ansprüchen 1 und 2, dadurch gekennzeichnet, daß eine Konzentration von 8—20 Gew.-% Alkalihydroxid gegeben ist.

4. Verfahren nach Ansprüchen 1 bis 3, dadurch gekennzeichnet, daß als Alkalilauge Natronlauge oder Kalilauge eingesetzt wird.

5. Verfahren nach Ansprüchen 1 bis 4, dadurch gekennzeichnet, daß die Trennung von Lösung und Bodenkörper bei − 15°C bis +35°C vorgenommen wird.

6. Verfahren nach Ansprüchen 1 bis 4, dadurch gekennzeichnet, daß die Trennung von Lösung und Bodenkörper bei −10°C bis +30°C vorgenommen wird.

7. Verfahren nach Ansprüchen 1 bis 6, dadurch gekennzeichnet, daß Mono-OD und DOD im eingesetzten Gemisch im Gewichtsverhältnis von 1 : 100 bis 100 : 1 vorliegt.

8. Verfahren nach Ansprüchen 1 bis 7, dadurch gekennzeichnet, daß Mono-OD und DOD im eingesetzten Gemisch im Gewichtsverhältnis von 5 : 100 bis 100 : 5 vorliegen.

9. Verfahren nach Ansprüchen 1 bis 8, dadurch gekennzeichnet, daß das Mono-OD und DOD enthaltende Gemisch zusätzlich Alkalisulfat und/oder Alkalisulfit enthält.

10. Verfahren nach Ansprüchen 1 bis 9, dadurch gekennzeichnet, daß man als Mono-OD und DOD enthaltendes Gemisch ein technisches, aus der Alkalischmelze der zugehörigen Diphenyl-sulfonsäuren stammendes Gemisch einsetzt.

## Claims

1. Process for the simultaneous recovery of 4-hydroxydiphenyl (mono-OD) and 4,4'-dihydroxydiphenyl (DOD) from a mixture containing the two substances in the free form or in the form of the alkali metal phenolates, characterised in that such a mixture is treated with an amount of aqueous alkali lye such that after formation of the alkali metal phenolates, where this is required, 1—15 mol of alkali metal hydroxide per mol of the diphenyl compounds are present and the concentration of alkali metal hydroxide is 4—25% by weight, relative to the amount of water present, the resulting solution, which essentially contains the DOD, is separated from the resulting solid phase, which essentially contains the mono-OD, at a temperature from −28°C to +40°C, and the solution and the solid phase are acidified separately in order to form the free mono-OD and the free DOD.

2. Prozess according to Claim 1, characterised in that 3—10 mol of alkali metal hydroxide are present per mol of diphenyl compound.

3. Process according to Claims 1 and 2, characterised in that the concentration of alkali metal hydroxide is 8—20% by weight.

4. Process according to Claim 1 to 3, characterised in that sodium hydroxide solution or potassium hydroxide solution is employed as the alkali lye.

5. Process according to Claims 1 to 4, characterised in that the separation of the solution and the solid phase is carried out at − 15°C to +35°C.

6. Process according to Claim 1 to 4, characterised in that the separation of the solution and the solid phase is carried out at −10°C to +30°C.

7. Process according to Claims 1 to 6, characterised in that mono-OD and DOD are present in a weight ratio of 1 : 100 to 100 : 1 in the mixture employed.

8. Process according to Claims 1 to 7, characterised in that mono-OD and DOD are present in a weight ratio of 5 : 100 to 100 : 5 in the mixture employed.

9. Process according to Claims 1 to 8, characterised in that the mixture containing mono-OD and DOD additionally contains alkali metal sulphate and/or alkali metal sulphite.

10. Process according to Claim 1 to 9, characterised in that an industrial mixture derived from the alkali melt of the corresponding diphenyl-sulphonic acids is employed as the mixture containing mono-OD and DOD.

## Revendications

1. Procédé de récupération simultanée de 4-hydroxydiphényle (mono-OD) et de 4,4'-dihydroxydiphényle (DOD) à partir d'un mélange contenant les deux substances sous la forme libre ou sous la forme des phénolates alcalins, caractérisé en ce qu'on traite ce mélange avec une quantité telle d'alcali caustique aqueux qu'après la formation éventuellement nécessaire des phénolates alcalins soient présentes 1 à 15 moles d'hydroxyde alcalin par mole des composés de diphényle et que soit établie une concentration de 4 à 25% en poids d'hydroxyde alcalin par rapport à la quantité de l'eau présente, à une température de −28°C à +40°C on sépare la solution obtenue, qui contient essentiellement le DOD, d'avec le corps obtenu sur le fond qui contient essentiellement le mono-OD, on sépare la solution d'avec le corps du fond et l'on acidifie pour la formation du mono-OD libre et du DOD libre.

2. Procédé selon la revendication 1, caractérisé en ce que sont présentes 3 à 10 moles d'hydroxyde alcalin par mole de composé de diphényle.

3. Procédé selon les revendications 1 et 2, caractérisé en ce qu'on établit une concentration de 8 à 20% en poids d'hydroxyde alcalin.

4. Procédé selon les revendications 1 à 3, caractérisé en ce qu'on utilise comme alcali caustique de la soude caustique ou de la potasse caustique.

5. Procédé selon les revendications 1 à 4, caractérisé en ce qu'on effectue la séparation de la solution d'avec le corps du fond à −15°C à +35°C.

6. Procédé selon les revendications 1 à 4, caractérisé en ce qu'on effectue la séparation de la solution d'avec le corps du fond à −10°C à +30°C.

7. Procédé selon les revendications 1 à 6, caractérisé en ce que le mono-OD et le DOD dans le mélange employé se présente dans le rapport pondéral de 1 : 100 à 100 : 1.

8. Procédé selon les revendications 1 à 7, caractérisé en ce que le mono-OD et le DOD dans le mélange employé se présentent dans le rapport pondéral de 5 : 100 à 100 : 5.

9. Procédé selon les revendications 1 à 8, caractérisé en ce que le mélange contenant le mono-OD et le DOD contient en plus du sulfate alcalin et/ou du sulfite alcalin.

10. Procédé selon les revendications 1 à 9, caractérisé en ce qu'on utilise comme mélange contenant du mono-OD et du DOD un mélange technique en provenance de la fusion alcaline des acides diphényl-sulfoniques apparentés.